# EUROPEAN PATENT SPECIFICATION

(11) **EP 2 163 648 B1**
(45) Date of publication and mention of the grant of the patent: **25.08.2010**
(21) Application number: 08164395.9
(22) Date of filing: 16.09.2008
(51) Int. Cl.: C12Q 1/68

(54) **PCR Ribotyping of C. difficile**
PCR-Ribotyping
Ribo-typage PCR

(43) Date of publication of application: 17.03.2010
(73) Proprietor: Österreichische Agentur für Gesundheit und Ernährungssicherheit GmbH, 1226 Vienna (AT)
(72) Inventor: Indra, Alexander, 1096 Vienna (AT)
(74) Representative: Sonn & Partner Patentanwälte

(56) References cited:
- WO-A-96/19585
- INDRA A ET AL: "First cases of Clostridium difficile PCR ribotype 027 acquired in Austria." EURO SURVEILLANCE : BULLETIN EUROPÉEN SUR LES MALADIES TRANSMISSIBLES = EUROPEAN COMMUNICABLE DISEASE BULLETIN 15 MAY 2008, vol. 13, no. 20, 15 May 2008 (2008-05-15), XP007906903 ISSN: 1560-7917
- BIDET P ET AL: "Comparison of PCR-ribotyping, arbitrarily primed PCR, and pulsed-field gel electrophoresis for typing Clostridium difficile." JOURNAL OF CLINICAL MICROBIOLOGY JUL 2000, vol. 38, no. 7, July 2000 (2000-07), pages 2484-2487, XP002481177 ISSN: 0095-1137
- BIDET P ET AL: "Development of a new PCR-ribotyping method for Clostridium difficile based on ribosomal RNA gene sequencing." FEMS MICROBIOLOGY LETTERS 15 JUN 1999, vol. 175, no. 2, 15 June 1999 (1999-06-15), pages 261-266, XP002481178 ISSN: 0378-1097
- STUBBS S L ET AL: "PCR targeted to the 16S-23S rRNA gene intergenic spacer region of Clostridium difficile and construction of a library consisting of 116 different PCR ribotypes." JOURNAL OF CLINICAL MICROBIOLOGY FEB 1999, vol. 37, no. 2, February 1999 (1999-02), pages 461-463, XP007906905 ISSN: 0095-1137
- GURTLER V ET AL: "Classification of medically important clostridia using restriction endonuclease site differences of PCR-amplified 16S rDNA." JOURNAL OF GENERAL MICROBIOLOGY NOV 1991, vol. 137, no. 11, November 1991 (1991-11), pages 2673-2679, XP001536960 ISSN: 0022-1287
- MITCHELSON KEITH R: "The use of capillary electrophoresis for DNA polymorphism analysis." MOLECULAR BIOTECHNOLOGY MAY 2003, vol. 24, no. 1, May 2003 (2003-05), pages 41-68, XP007906915 ISSN: 1073-6085

## Description

The present invention relates to a method of polymerase chain reaction (PCR) analysis of polymorphisms in the 16s-23s intergenic spacer region of Clostridium difficile ("PCR ribotyping of Clostridium difficile").

Clostridium difficile (C. difficile) is the most frequently identified cause of hospital-acquired diarrhea and is the causative agent of pseudomembranous colitis. A new emerging hyper-virulent strain of C. difficile - PCR ribotype 027 - causes more severe disease and is associated with a higher mortality rate than other types (Kuijper et al., 2006). This increased virulence is associated with a defect in a toxin regulator gene resulting in hyperproduction of toxins A and B. The incidence of C. difficile-associated disease (CDAD) due to type 027 is increasing in the USA, Canada, Asia and Europe (Kuijper et al., 2006). However, other PCR ribotypes are currently also increasing, such as type 106 in the UK and type 078 in the Netherlands. Correct strain characterization is of utmost importance to recognize new circulating and emerging types.

There are several ways known and applied of characterisation and typing of C. difficile, such as PCR ribotyping, arbitrarily primed PCR (AP-PCR), amplified fragment length polymorphism (AFLP), pulsed-field gel electrophoresis (PFGE), multilocus sequence typing (MLST), and restriction endonuclease analysis (REA), repeat-element-PCR (REP) and multilocus variable-number tandem-repeat (VNTR) analysis (MLVA).

PCR ribotyping is a PCR targeted to the 16S-23S rRNA gene intergenic spacer region of C. difficile (WO 96/19585 A, Stubbs et al., 1999; currently applied standard reported in Bidet et al., 1999). PCR ribotyping as well as AP-PCR are regarded as being techniques not being able to provide sufficient discriminatory power to distinguish among the circulating genotypes, which is believed to obscure epidemiologic investigations (Marsh et al., 2006). In addition, AP-PCR is suspected to suffer from lack of reproducibility.

PFGE is also not a consistently reliable subtyping tool for C. difficile due to DNA degradation of some strains, resulting in frequent genotyping failures, and studies comparing AFLP with PFGE have demonstrated that AFLP is a more reliable C. difficile genotyping tool than PFGE.

The application of REA for studying the epidemiology of C. difficile (Kuijper et al., 1987; Gürtler et al., 1991) is considered a standard in C. difficile genotyping and has been instrumental in describing outbreak strains, including the current REA BI clone that has been found in multiple hospitals in North America and parts of Europe. However, the method is not readily transportable between laboratories, since interpretation of REA banding patterns is subjective and confirmation of REA matches requires analysis of isolates on the same gel. Nevertheless, this method is still applied on a regular basis in the U.S.

REP-typing has been extensively analysed in a multicenter evaluation for possible interlaboratory exchange of data but has been regarded as not being suitable for standardisation due to the fact, that this technique showed in many laboratories a low discrimination and reproducibility (Deplano et al., 2000).

A genotyping method that is amenable to C. difficile surveillance and the generation of a C. difficile genotypic database is necessary to efficiently track transmission of the organism both locally and globally. Multilocus variable-number tandem-repeat (VNTR) analysis (MLVA) has been regarded as a useful genotyping tool that has demonstrated application to epidemiologic studies and outbreak detection for a variety of bacterial organisms. MLVA is regarded as a highly discriminatory genotyping method and proposed as "a new tool for subtyping of newly emerging variants of C. difficile" (van den Berg et al., 2007). It was therefore widely proposed that a new standardised protocol for routine epidemiological discrimination of C. difficile should be based on MLVA (Marsh et al., 2006; Supply et al., 2006).

It is clear that it is necessary to develop a method for identification and analysis of C. difficile that allows standardization and interlaboratory exchange of data, specifically for fast and reproducible epidemiologic investigations of nonsocomial transmission and typing differentiation. This method must also be robust and reliable so that testing of a large number of specimen is possible in different locations.

Therefore, according to the present invention a method of polymerase chain reaction (PCR) analysis of polymorphisms in the 16s-23s intergenic spacer region of Clostridium difficile ("PCR ribotyping of Clostridium difficile") comprising the use of a 16s primer corresponding to bases 1450 to 1501 of the 16S ribosomal RNA gene of C. difficile and a 23s primer corresponding to bases 1 to 50 of the 23S ribosomal RNA gene of C. difficile, is provided, wherein the PCR is performed with a labeled 16s primer and an unlabeled 23s primer and the DNA molecules provided by the PCR are separated by size using capillary gel electrophoresis.

During the investigations for the present invention, it surprisingly turned out that the PCR ribotyping method for C. difficile, which has been regarded as being finally optimised (Bidet et al., 1999; Indra et al., 2008) - but finally not suitable for inter-laboratory standardisation (Marsh et al., 2006) - , can be made more sensitive by using a specifically labeled primer set in the PCR and by analysing the PCR fragments being produced from the 16s-23s intergenic spacer region of C. difficile by capillary gel electrophoresis methods. This allows a significantly improved way for analysing and genotyping C. difficile isolates.

First, it surprisingly turned out that labeling of the primers is of paramount importance for obtaining a reproducible and reliable peak pattern. According to the present invention, the PCR is performed with a labeled 16s primer and an unlabeled 23s primer. It was observed that with traditional labeling (of the 23s primer) according to Bidet et al., 1999, non-exploitable double peak patterns are obtained with a critical bp distance (of e.g. 3bp peak distance). Due to the frequently occurring regular PCR products with high similarity, data could not be reliably interpreted, not even with capillary gel electrophoresis, because resolution of capillary gel electrophoresis is reported to be 1 to 1,5 bp (Chen et al., 1992).

With the capillary gel electrophoresis based PCR ribotyping according to the present invention, the problem of hampered inter-laboratory comparing of typing results is overcome, while at the same time the hands-on time for PCR ribotyping is substantially diminished. This leads to a substantial improvement of a laboratory's capacity for C. difficile PCR ribotyping. The capillary gel electrophoresis used according to the present invention has a resolution which allows distinguishing fragments with a length difference of 2b, preferably of 1,5b, especially of 1b The electrophoresis should be able to separate fragments with 150 to 650b in length. Preferably, a size standard is run in the electrophoresis to exactly define the size of the fragments. This electrophoresis technique analyses single strand DNA.

PCR ribotyping for C. difficile is in principle a well established methodology, especially in Europe. This technique was first described by Stubbs (Stubbs et al., 1999) and further developed and standardised with respect to primers, PCR details, etc. by Bidet et al. (Bidet et al., 2000). Background to the method is described i.a. by Sadeghifard et al., 2006. At least 150 different PCR ribotypes have been recognized, but only a few of them are known as human enteropathogens. Agarose gel based PCR ribotyping has evolved as the most widely used method in European reference laboratories, hampered only by the lack of standardized type strain collections, which have led to various nomenclatures (Kato et al., 2001; Spigaglia et al., 2004; Aspevall et al., 2006).

With the present invention it was e.g. possible to divide isolates belonging to a certain PCR ribotype according to the established system (type 014) into seven (!) subgroups. The present invention therefore overcomes the drawbacks reported for PCR ribotyping in the field at present (e.g. Marsh et al., 2006). Therefore, in contrast to the comments by Marsh et al. to the state-of-the-art PCR ribotyping for C. difficile, the PCR ribotyping technology according to the present invention shows sufficient discriminatory power to distinguish among the circulating genotypes and is, accordingly, suitable for epidemiologic investigations.

Capillary gel electrophoresis is an established method in the field of molecular biology, especially in sequencing technology, and therefore easily to be adapted by the skilled man in the art to the present invention. However, the fact that this technology is applicable with advantageous results (especially with respect to standardisation benefits) in PCR ribotyping of C. difficile was evidently surprising, as the developments in C. difficile typing standardisation efforts clearly went into completely different directions. There is ample teaching available for the general teaching for capillary gel electrophoresis (e.g. Luckey et al., 1990; Mansfield et al., 1996; Mitchelson, 2003).

The PCR ribotyping is preferably performed according to the standardised protocol of Bidet et al., 1999 (see also: Sadeghifard et al., 2006; Indra et al., 2008), however, with the essential difference that the 16s primer is the only labeled primer. In this method, primers corresponding to bases 1482-1501 of the 16S rRNA gene ("16s primer") and 1-24 of the 23S rRNA gene ("23s primer") of C. difficile are used. It is preferred in any way to use such primers or analogous primers for the PCR ribotyping according to the present invention. The primer pair corresponding to bases 1450 to 1501 of the 16S ribosomal RNA gene of C. difficile on the one side and corresponding to bases 1 to 50 of the 23S ribosomal RNA gene of C. difficile on the other side is the PCR primer pair according to the invention, regardless whether the one or the other is used as forward primer. The term "corresponding" in this connection means that the primers used should bind to the region defined by the bases 1450 to 1501 of the 16S rRNA gene ("16s primer") and the bases 1 to 50 of the 23S rRNA ("23s primer"), respectively (or to the complementary sequence, respectively). For example, the "Bidet" primers (1482-1501 of the 16S rRNA; 1-24 of the 23S rRNA: 5'-GTGCGGCTGGAT-CACCTCCT and 5'-CCCTGCACCCTTAATAACTTGACC) or the "Sadeghifard" primers (1477-1493 of the 16S rRNA gene and 21-41 of the 23S rRNA) are suitable. In general, one primer from region 4 and one primer from region 5 (Sadeghifard et al., 2006) may be applied. It is advantageous to perform the PCR reaction according to the present invention with a reduced number of cycles compared to the established PCR ribotyping methods using agarose gels. Whereas for the prior art methods 30 to 35 cycles are used, according to the present invention preferably less than 30 cycles are used, e.g. 18 to 28, especially 20 to 25 (for example 21, 22 or 23 cycles). With this reduced number of cycles, the performance of the method as a whole further improves. In contrast to the method of Bidet et al. 1999, the PCR is performed with a labeled 16s primer and an unlabeled 23s primer and the fragment analysis is performed by capillary gel electrophoresis in the method according to the present invention.

The labeling of the 16s primer is preferably performed at the 5' end of the oligonucleotide, for example within the first five 5' nucleotides, preferably within the first three 5' nucleotides, especially at the 5' nucleotide. However, it is also possible to label the primer internally, as long as the label does not interfere with the PCR. Preferably, labeling is performed with labels established in PCR, especially labeling with fluorescent dyes, especially with carboxyfluorescein (FAM, hexachlorofluorescein (HEX), tetrachlorofluorescin (TET), N,N,N',N'-tetramethyl-6-carboxyrhodamin (TAMRA), VIC, NED or 5-carboxy-X-rodamine (ROX).

The present invention relates to the use of a method according to the present invention for the analysis of C. difficile samples, especially of samples from diarrhea patients, especially of a plurality of such patients. As mentioned above, the present method is optimal for fast and reliable typing of C. difficile, especially in case of epidemiologic studies. Specifically, the present method is suited for emergency cases where a large number of patient's samples have to be analysed and typed within a very short time.

According to another aspect, the present invention relates to a kit specifically designed to perform the present invention and comprising a PCR kit for analysis of polymorphisms in the 16s-23s intergenic spacer region of C. difficile and a capillary gel electrophoresis apparatus, wherein the PCR kit contains a 16s-primer corresponding to bases 1450 to 1501 of the 16S ribosomal RNA gene of C. difficile and a 23s primer corresponding to bases 1 to 50 of the 23S ribosomal RNA gene of C. difficile, characterized in that the 16s primer is labeled and the 23s primer is unlabeled. The PCR kit contains suitable primers as defined above and all components necessary to perform the PCR, such as polymerase, nucleotide mixes, buffers, etc.. The kit also comprises a PCR thermocycler to perform the PCR.

The invention is further illustrated by means of the following examples and the drawing figures, yet without being restricted thereto.

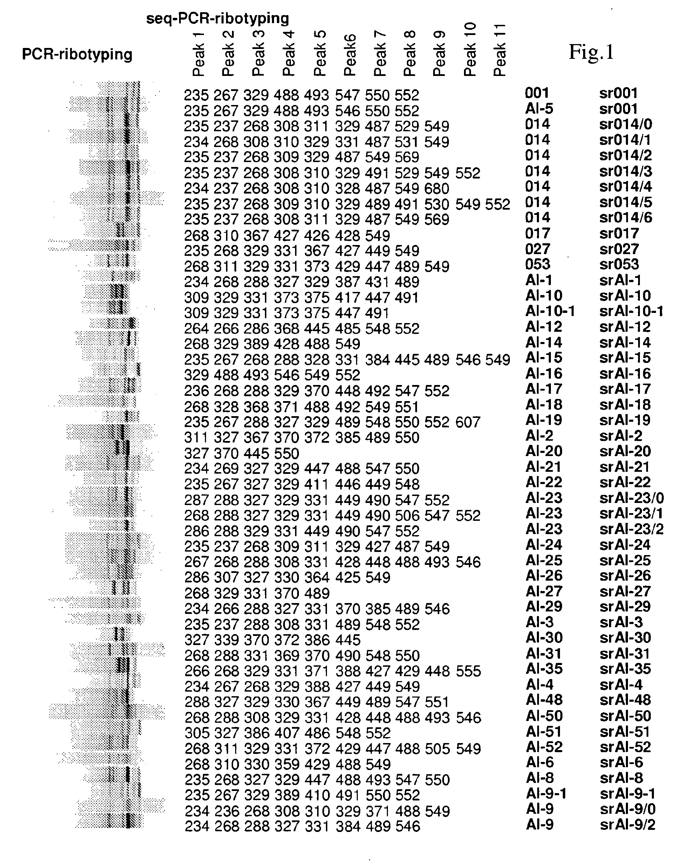

Fig. 1 shows ribotype patterns found by classical agarose based gel electrophoresis (left column) in comparison with the capillary gel electrophoresis based method (column labeled "seq-PCR3 ribotyping"); PCR ribotypes resulting from capillary gel electrophoresis received the prefix "sr".

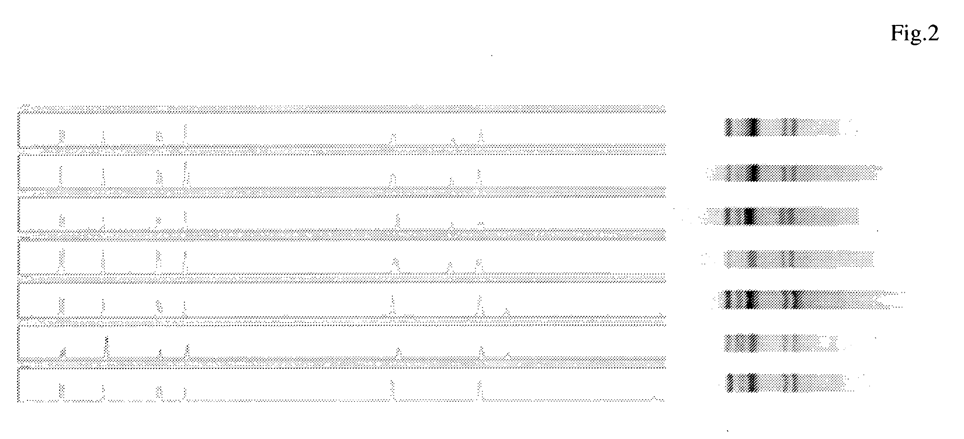

Fig. 2 shows capillary gel electrophoresis chromatograms of 7 seven isolates of ribotype 014 showing seven distinct subgroup patterns (left); patterns produced by classical agarose based gel electrophoresis PCR ribotyping (right); lane 1 shows the chromatogram (left) and PCR ribotype pattern (right) of the reference-strain for ribotype 014.

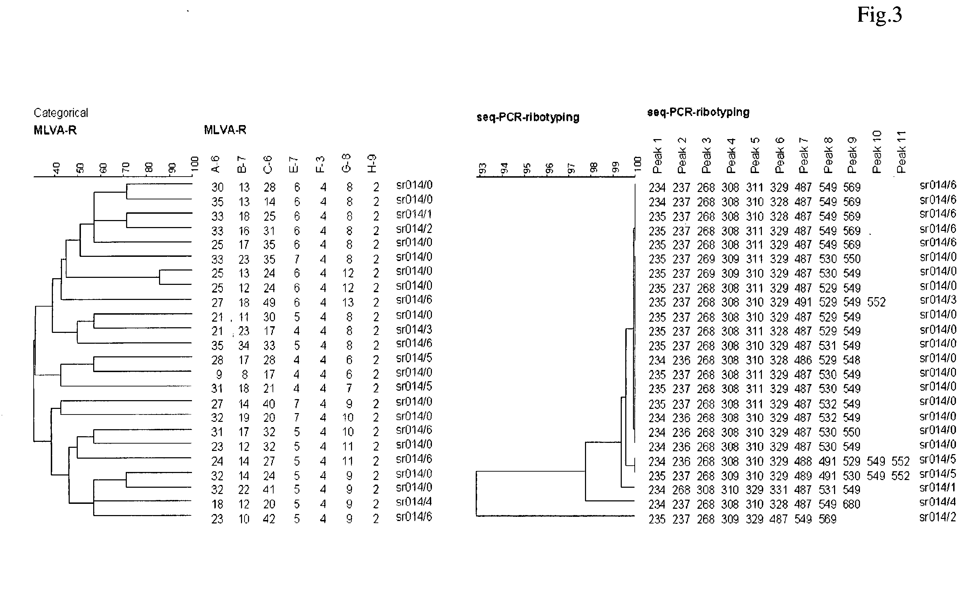

Fig. 3 shows MLVA and capillary gel electrophoresis based PCR ribotyping of 24 isolates 1 belonging to ribotype 014; capillary gel electrophoresis based ribotyping yielded seven different 014 subgroups, shown on the right side and assigned names sr014/0 to sr014/6; MLVA subtyping (left) of the isolates yielded three unrelated subgroups, without obvious correlation to sr-subgroups.

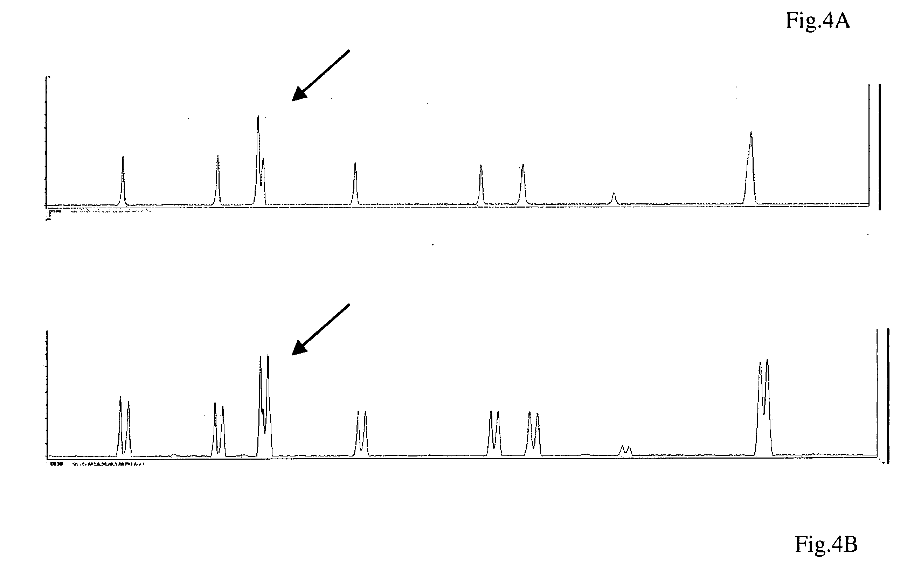

Fig. 4 shows the PCR peak pattern with the labeled 16s primer (A) and with the labeled 23s primer (B).

### EXAMPLES

### Materials and Methods

### Isolates

A total of 146 C. difficile isolates were studied. One-hundred-forty-and-one isolates were collected during a period from February 2006 till January 2007 from patients of 25 Austrian healthcare facilities as described elsewhere (Indra et al.,2008). Control strains each of C. difficile ribotypes 001, 014, 017, and 027 were obtained from the Department for Medical Microbiology, University Medical Center, Leiden. The control strain ribotype 053 was an Austrian isolate ribotyped by Jon Brazier (Department of Microbiology, University of Cardiff, Wales, UK).

### Agarose gel electrophoresis based ribotyping

Classical agarose gel based PCR ribotyping was performed with primers 16s (5'-GTGCGGCTGGATCACCTCCT) and 23s (5'-CCCTGCACCCTTAATAACTTGACC) (VBC12 Biotech, Vienna, Austria) as described by Bidet et al. (1999). Briefly, DNA extraction from cultures to a final volume of 50 µl was done using the MagnaPur-Compact (Roche, Mannheim, Germany) according to the manufacturer's recommendations. Twenty-five µl HotStar Taq master mix (Quiagen, Hilden, Germany) was used with 1 µl (10 pmol/µl) of each primer, 18 µl water and 5 µl DNA. Amplification was done with a commercial PCR-thermocycler running a 15 min 95°C initial enzyme activation, 35 cycles of 1 min at 95°C for denaturation, 1 min at 57°C for annealing, and 1 min at 72°C for elongation and a 5 min 72°C final elongation step. The amplified products were separated by electrophoresis on 1.5% agarose (Bio-rad, Hercules, CA, USA) for 4 h at 100 V using a 100 to 1000 bp ladder (Fermentas, Burlington, Canada) as size standard every 10th lane.

Ribotype patterns different from those of the five reference strains available to the Austrian C. difficile reference laboratory (001, 014, 017, 027, 053) were previously labeled with consecutive numbers and the prefix AI (Indra et al., 2008). Strains included in this study belonged to ribotypes 001 (n=1); 014 (n = 25), 017 (n=2), 027 (n=2), 053 (n = 24), AI-1 (n=3), AI-2 (n=4), AI-3 (n=2), AI-5 (n = 26), AI-6 (n=9), AI-8 (n=2), AI-9 (n=4), AI-10 (n=3), AI-14 (n=7), AI-17 (n=2), AI-19 (n=2), AI-23 (n=5), AI-50 (n=2), and one each of AI-4, AI-9-1, AI-10-1, AI-12, AI-15, AI-16, AI 18, AI-20, AI-21, AI-22, AI-24, AI-25, AI-26, AI-27, AI-29, AI-30, AI-31, AI-1 35, AI-48, AI-51, and AI-52.

### Capillary gel electrophoresis based ribotyping

Capillary gel electrophoresis based PCR ribotyping was performed with primers 16s (5'-GTGCGGCTGGATCACCTCCT) and 23s (5'-CCCTGCACCCTTAATAACTTGACC) as described Bidet et al. (Bidet et al., 2000). The 16s primer was labeled at the 5' end with either carboxyfluorescein (FAM), hexachlorofluorescein (HEX) or tetrachlorofluorescin (TET). The 23s primer was not labeled (except in the comparative experiment) DNA used for gel based ribotyping was diluted 1:20. Twenty-five µl HotStar Taq master mix (Quiagen, Hilden, Germany) was used with 0.3 µl (10 pmol/µl) of each primer, 20.7 µl water and 1.5 µl DNA. Amplification was done with a commercial PCR thermocycler running a 15 min 95°C initial enzyme activation, 22 cycles of 1 min at 95°C for denaturation, 1 min at 57°C for annealing, and 1 min at 72°C for elongation and a 30 min 72°C final elongation step. The PCR fragments were analyzed using an ABI 310 (Applied Biosystems, Foster City, USA) genetic analyser, with a 50-625bp TAMRA ladder (Chimerx, Wisconsin, USA) as an internal marker for each sample. A 41 cm capillary loaded with a POP4 gel (Applied Biosystems, Foster City, USA) was employed for this. Injection was done with 5kV over 5sec, with a total running time of 28 min at 15 kV run voltage. The size of each peak was determined by Peakscanner software 1.0 (Applied Biosystems, Foster City, USA). Ribotype patterns generated by capillary gel electrophoresis were named using the prefix "sr". For tests on reproducibility a second master mix (Ampli-Taq-Gold master mix; Applied Biosystems, Foster City, USA) was employed.

### MLVA typing

Multiple-locus variable-number tandem-repeat analysis (MLVA) was done using the method described by van den Berg et al. (van den Berg et al., 2007). Briefly, locus A6, B7, C6, E7, F3, G8 and H9 were amplified using fluorescence labeled primers. The repeats were amplified using a single PCR protocol. The amplification reactions were performed in a final 1 volume of 20 µl containing 10 µl of HotStart Aplitic-Gold master mix (Applied Biosystems, Foster City, USA), 1 µM of each primer, and 1 µl of DNA. After an initial enzyme activation step of 5 min at 95°C, the protocol consisted of 35 cycles of 30 s at 94°C for denaturation, 30 s at 50°C for annealing, and 30 s at 72°C for elongation. A final elongation step was performed for 30 min at 68°C. The forward primers were labeled at the 5' end with either carboxyfluorescein (FAM), hexachlorofluorescein (HEX), or tetrachlorofluorescein (TET). PCR fragments were analyzed using multicolored capillary gel electrophoresis on an ABI310 genetic analyzer, with a TAMRA500 ladder as an internal marker for each sample. The size of each peak was determined by Peakscanner software 1.0 (Applied Biosystems, Foster City, USA). Cluster-analysis was done using Bionumerics software Version 5.0 (Applied Maths, Sint-Martens-Latem, Belgium), by applying UPGMA categorical analysis.

### Agarose gel DNA extraction

DNA fragments were eluted out of agarose gels using the MinElute Gel Extraction Kit (Quiagen, Hilden, Germany) according to manufacturer's instructions.

### Data analysis

Data concerning PCR ribotype band sizes and MLVA were analyzed using Bionumerics software Version 5.0 (Applied Maths, Sint-Martens-Latem, Belgium). The repeat numbers were analyzed using BioNumerics, version 3.5, software (Applied Maths, Kortrijk, Belgium) and the unweighted pair group method (UPGMA) with arithmetic averages with the multistate categorical similarity coefficient (MCSC). All markers were given an equal weight, irrespective of the number of repeats. Subsequently, if two strains had an equal number of repeats in five of seven markers, they are 72% and considered to be closely related.

Fragment size was calculated using Bionumerics software for agarose based electrophoresis and Peakscanner software for capillary gel electrophoresis. Results from capillary gel electrophoresis based PCR ribotyping were imported into Bionumerics 5.0. Peaks were counted as bands when they showed at least 10% of the height of the highest peak of the individual run. Doublepeaks were only counted if they were separated by more than 2bp. Cluster-analysis was done applying a UPGMA pearson correlation using Bionumerics 5.0 software.

### Web database

A web based database (http://webribo.ages.at) was created for capillary gel electrophoresis based PCR ribotyping results. For sr-types with more than two entries for individual isolates an artificially calculated reference strain was created consisting of the mean fragment size values of the corresponding peaks of the same size. An error margin of +/- 4 b was incorporated in the analysis algorithm of the database.

With a web based application every user is able to enter his/her data after registration and receives a ribotype name if his/her isolate is of a known type. Any unknown PCR ribotype patterns will be accepted if their isolate's chromatogram is submitted to the database administrator for proof of quality.

### Comparative experiment

In a comparative experiment, only the 23s primer was labeled with fluorescent dye (as done in the previous experiments for the 16s primer) and compared in a PCR to the results of a PCR set up with the labeled 16s primer.

### RESULTS

Capillary gel electrophoresis yielded up to 11 fragments per isolate. Fragments had a minimum fragment size of 233 b and a maximum fragment size of 680 b. Between isolates of one ribotype, the differences of minimal and maximal fragment size found with conventional agarose based gel electrophoresis were as high as 27 b for certain fragments. Capillary gel electrophoresis based results for individual peaks within one ribotype did not exceed deviations of more than one single base pair. Figure 1 compares the 47 ribotypes resulting from capillary gel electrophoresis based PCR ribotyping with the 39 ribotypes generated by classical agarose gel electrophoresis based ribotyping. All but one of the 5 PCR ribotypes from reference strains were clearly reflected by the results of capillary based typing.

Capillary gel electrophoresis based PCR ribotyping divided ribotype 014 (n= 24) into seven distinct subgroups as shown in Fig. 2. Using a similarity index of 72%, MLVA was capable to differentiate at least 20 subtypes (Fig 3).

To calculate reproducibility, seven isolates (one for each subgroup of PCR-ribotype 014) were tested in parallel using three different 16s primers (labeled with FAM, TET, and HEX, respectively) and two different master mixes (Hotstar-master mix, and Ampli-Taq-Gold master mix, respectively). Results of capillary gel electrophoresis based PCR ribotyping were highly reproducible, independent from the brand of reagent used. The standard deviation per peak was +/- 0.5 b with a median upper confidence interval of 0.83.

For all 26 isolates of ribotype AI-5, an agarose gel band of 360 to 390 b size was not reflected in the chromatograms of capillary gel electrophoresis. Three AI-5 isolates were amplified with a fluorescence labeled forward primer. The 360 to 390 b fragment was eluted out of the agarose and purified DNA was submitted to capillary gel electrophoresis separation. Again, the band of 360 to 390 b size could not be found in the capillary gel electrophoresis.

Capillary gel electrophoresis of this purified DNA instead yielded three fragments of 328 b, 234 b and 266 b size. Fragments of these three sizes can be found with both methods (i.e. with classical and with capillary based typing) in all 26 AI-5 and in 1 the 001 isolate. Capillary gel electrophoresis based PCR-ribotyping yielded the following 47 Austrian ribotype patterns for the 146 tested isolates: sr001 (n=28), sr014/0 (n= 14), sr014/5 (n=2), sr014/6 (n=5), sr017 (n=2), sr027 (n=2), sr053 (n=23), srAI-1 (n=3), srAI-2 (n=4), srAI-3 (n=2), srAI-6 (n=9), srAI-8 (n=2), srAI-9-1 (n=2), srAI-10 (n=3), srAI-14 (n=7), srAI-17 (n=2), srAI-19 (n=2), srAI-23/2 (n=3), srAI-50 (n=3), and one each of sr014/1, sr014/2, sr014/3, sr014/4, srAI-10-1, srAI-12, srAI-15, srAI-16, srAI-18, srAI-20, srAI-21, srAI-22, srAI-23/0, srAI-23/1, srAI-24, srAI-25, srAI-26, srAI-27, srAI-29, srAI-30, srAI-31, srAI-35, srAI-4, srAI-48, srAI-51, srAI-52, srAI-9/0, srAI-9/2.

The comparative experiment showed that only labeling of 16s primers resulted in reliable and reproducible results. In the comparative experiment it is clearly visible that only the PCR using the labeled 16s primer yielded reliable and reproducible results which allows routine testing also on a large number of samples. With a labeled 23s primer, double peak patterns occur which make the analysis not utilisable for routine testing or even not usable at all. The results of the comparative experiment are shown in Fig.4. In Fig 4A the experiment with the labeled 16s primer is shown; Fig 4B shows the results of the PCR with the labeled 23s primer. In both figures, the frequently occurring large regular PCR products being similar in size are exemplary highlighted by the arrow. From such highly similar PCR products, data interpretation is usually difficult also bearing in mind that the resolution of capillary gel electrophoresis is in the range of 1 to 1,5 b (Chen et al., 1992). In contrast to the double peak structure obtained with the labeled 23s primer, no double peak formation is obtained by using the labeled 16s primer.

The problem concerning usability of the electrophoresis pattern is specifically evident at the peak highlighted by the arrow in both figures: At this position, two "regular" peaks are close to each other. With the 23s labeled primer, double peaks occur which - at this position - fuse into a peak conglomerate of three peaks, although actually 4 peaks should be visible (two double peaks): The second peak (at about 329,5 b) of the first fragment falls together with the first peak of the second fragment (at about 330,5 b). These fragments are only separated by 1 to 1,5 p in length. Accordingly, there is the risk that these two fragments are either measured as a single peak or as two peaks, depending on slightly differing differences in running conditions of the electrophoresis. This double peak phenomenon only occurs with the labeled 23s primers, not with the labeled 16s primers.

### DISCUSSION

PCR ribotyping patterns are based on size variations in the 16s-23c intergenic spacer regions of the bacterial rRNA (rrn) operon. According to Sadeghifard et al. the size of these regions ranges from 238 to 566 b, which compares well to the fragment sizes found in the present study (233 - 680 b) (Sacieghifard et al., 2006). Although the absence of an international type strain collection has lead to different nomenclature schemes, PCR-ribotyping has evolved as the standard typing method for C. difficile in Europe (Aspevall et al., 2006; Kato et al., 2001). PCR ribotyping has proven to be a valuable tool for epidemiological investigations of C. difficile outbreaks, although certain PCR ribotypes can be divided in several subtypes using other molecular typing methods.

A capillary gel electrophoresis based subtyping assay was developed, which significantly diminishes the hands on time required for C. difficile PCR ribotyping. The results were highly reproducible, independent of reagent-batches or reagent-brands used. Of the 5 PCR ribotypes from reference strains tested, all but one ribotype were clearly reflected in the results of capillary based typing. Capillary gel electrophoresis divided PCR ribotype 014 into seven subgroups, a finding that may reflect the existence of previously unrecognised new ribotypes or mere over-discrimination. MLVA results did not underpin the existence of these seven sr-subgroups for 014. By using a web based software program, we are able to correctly identify 014 by simply allocating the 7 subgroup patterns to one ribotype, i.e. to PCR ribotype 014. This database (http://webribo.ages.at) also allows smooth data comparison between laboratories, similarly as the spa-typing web database (http://spaserver.ridom.de) does for Staphylococcus aureus.

Capillary gel electrophoresis typing showed that the Austrian PCR ribotype AI-5 is identical to PCR ribotype 001, despite a one band difference in classical agarose gel electrophoresis. All A1-5 isolates (n=27) had an identical pattern. The organization of the rrn-operon might explain the extra band formation of the 27 isolates of AI-5 (Sadeghifard et al., 2006). While in agarose gel based electrophoresis double stranded DNA fragments are separated, capillary gel electrophoresis separates denatured single stranded DNA, stabilized with foramid. Sadeghifard et al. showed that the 16-23s intergenic spacer region has a mosaic nature, like the whole genome of C. difficile itself (Sadeghifard et al., 2006). After extraction of this extra band from the agarose gel, there were three fragments of different sizes found in the chromatogram of capillary gel electrophoresis (Fig. 2). This band of the Austrian ribotype AI-5 is in fact a product of wrong hybridization of different 16s-23s intergenic spacer regions of similar sequence. By analyzing the sequences of 55 different 16s-23s intergenic spacer regions, Sadeghifard et al., previously showed that isolates of the same ribotype can have different intergenic spacer region sequences (Sadeghifard et al., 2006).

Capillary gel electrophoresis results also indicate, that the two designated Austrian ribotypes AI-9 and AI-23 can not be considered unique ribotypes. In hindsight, both must be considered incorrect subtyping results caused by the low discrimination of classical agarose gel electrophoresis. Such findings emphasizes the importance of the establishment of a European reference strain collection, as a prerequisite for establishment of a common nomenclature. Conventional agarose gel based PCR ribotyping is easy to use and relatively cheap, but is hampered by a poor resolution concerning fragment length analysis. By using 5' fluorescent labeled forward primers for amplification of the spacer regions, PCR ribotyping was established as a capillary gel electrophoresis based assay by the present invention. The new capillary based assay allows inter-laboratory exchange of data without the need of cumbersome standardization of equipment, reagents, and operating procedures. For institutes equipped with a capillary sequencer, the method reduces the cost of PCR ribotyping by drastically diminishing the hands-on time to about one-third when compared to the conventional agarose gel based method.

PCR ribotyping presently is the preferred typing method for Clostridium difficile in Europe. Capillary gel electrophoresis based PCR ribotyping is a proper way for overcoming the problem of hampered inter-laboratory comparing of typing results while at the same time substantially improving a laboratory's capacity for C. difficile PCR ribotyping.

Labeling of 16s primers prevents the occurrence of double peaks. In contrast to the PCR with labeled 23s primers (as performed by Bidet at al., 1999, 2000) where double peaks occur for each of the fragments, PCR with labeled 16s primers results in single peaks which allow an accurate separation of fragments with similar size, i.e. a size difference of only 1 or 2 b. This separation performance is essential for applying the method on a routine basis in serial testing. Therefore, the method according to the present invention is also easily subjected to standardisation and automatisation and allows reliable testing in different laboratories.

### REFERENCES:

Aspevall et al., Antimicrob Agents Chemother 50(2006): 1890-1892.
Bidet et al., FEMS Microb. Lett. 175 (1999): 261-266.
Bidet et al.(2), J. Clin.Microbiol. 38 (2000): 2484-2487.
Chen et al., NAR 20 (1992): 4873-4880.
Deplano et al., J. Clin. Microbiol. 38 (2000): 3527-3533.
Gürtler et al., J. Gen. Microbiol. 137 (1991): 2673-2679.
Indra et al., J. Med. Microbiol. 57 (2008): 1377-1382.
Indra et al., Eur. Comm. Dis. Bull. 13 (2008), ISSN: 1560-7917.
Kato et al., J. Clin. Microbiol. 39 (2001): 1391-1395.
Kuijper et al., J. Clin. Microbiol. 25 (1987): 751-753.
Kuijper et al., Clin. Microbiol. Infect. 12 (2006) Suppl 6: 2-18.
Luckey et al., Nuc. Acids Res.18 (1990): 4417-4421;
Mansfield et al., Genome Res. 6 (1996): 893-903;
Marsh et al., J. Clin. Microbiol. 45 (2007): 1024-1028.
Mitchelson, Mol. Biotech. 24 (2003): 41-68.
Sadeghifard et al., Appl. Environ. Microbiol. 72 (2006):7311-7323.
Spigaglia et al., J. Med. Microbiol. 53 (2004): 1129-1136. Stubbs et al., J. Clin. Microbiol 3(1999): 461-463.
van den Berg et al., J. Clin. Microbiol. 45 (2007): 1024-1028. WO 96/19585 A

### SEQUENCE LISTING

<110> OSTERREICHISCHE AGENTUR FÜR GESUNDHEIT UND ERNÄHRUNGSSICHERHEIT GMBH
<120> PCR ribotyping
<130> R52800
<160> 2
<170> PatentIn version 3.4
<210> 1
   <211> 20
   <212> DNA
   <213> Clostridium difficile
<400> 1
   gtgcggctgg atcacctcct 20
<210> 2
   <211> 24
   <212> DNA
   <213> Clostridium difficile <400> 2
   ccctgcaccc ttaataactt gacc 24

## Claims

1. Method of polymerase chain reaction (PCR) analysis of polymorphisms in the 16s-23s intergenic spacer region of Clostridium difficile ("PCR ribotyping of Clostridium difficile") comprising the use of a 16s primer corresponding to bases 1450 to 1501 of the 16s ribosomal RNA gene of C. difficile and a 23s primer corresponding to bases 1 to 50 of the 23s ribosomal RNA gene of C. difficile, **characterized in that** the PCR is performed with a labeled 16s primer and an unlabeled 23s primer and that the DNA molecules provided by the PCR are separated by size using capillary gel electrophoresis.

2. Method according to claim 1, **characterized in that** PCR is performed with primers 5'-GTGCGGCTGGATCACCTCCT and 5'-CCCTGCACCCTTAATAACTTGACC.

3. Method according to claim 1 or 2, **characterized in that** the PCR is performed in less than 30 cycles, preferably 18 to 28 cycles, especially 20 to 25 cycles.

4. Method according to any one of claims 1 to 3, **characterized in that** the 16s primer is fluorescently labeled, preferably with carboxyfluorescein (FAM, hexachlorofluorescein (HEX), tetrachlorofluorescin (TET), N,N,N',N'-tetramethyl-6-carboxyrhodamin (TAMRA), VIC, NED or 5-carboxy-X-rodamine (ROX).

5. Use of a method according to any one of claims 1 to 4 for the analysis of C. difficile samples, especially of samples from diarrhea patients, especially of a plurality of such patients.

6. Kit comprising a PCR kit for analysis of polymorphisms in the 16s-23s intergenic spacer region of C. difficile and a capillary gel electrophoresis apparatus, wherein the PCR kit contains a 16s primer corresponding to bases 1450 to 1501 of the 16s ribosomal RNA gene of C. difficile and a 23s primer corresponding to bases 1 to 50 of the 23s ribosomal RNA gene of C. difficile, **characterized in that** the 16s primer is labeled and the 23s primer is unlabeled.

## Patentansprüche

1. Verfahren zur Polymerase-Kettenreaktions-Analyse (PCR-Analyse) von Polymorphismen in der 16s-23s "intergenic spacer"-Region von Clostridium difficile ("PCR Ribotyping von Clostridium difficile") umfassend der Verwendung eines 16s Primers entsprechend den Basen 1450 bis 1501 des 16s ribosomalen RNA-Gens von C. difficile und eines 23s Primers entsprechend den Basen 1 bis 50 des 23s ribosomalen RNA-Gens von C. difficile, **dadurch gekennzeichnet, dass** die PCR mit einem markierten 16s Primer und einem nicht markierten 23s Primer durchgeführt wird und, dass die durch die PCR bereitgestellten DNA-Moleküle unter Verwendung von Kapillargelelektrophorese nach Größe separiert werden.

2. Verfahren nach Anspruch 1, **dadurch gekennzeichnet, dass** die PCR mit den Primern 5'-GTGCGGCTGGATCACCTCCT und 5'-CCCTGCACCCTTAATAACTTGACC durchgeführt wird.

3. Verfahren nach Anspruch 1 oder 2, **dadurch gekennzeichnet, dass** die PCR in weniger als 30 Zyklen durchgeführt wird, vorzugsweise 18 bis 28 Zyklen, insbesondere 20 bis 25 Zyklen.

4. Verfahren nach einem der Ansprüche 1 bis 3, **dadurch gekennzeichnet, dass** der 16s Primer fluoreszierend markiert ist, vorzugsweise mit Carboxyfluorescein (FAM), Hexachlorofluorescein (HEX), Tetrachlorofluorescein (TET), N,N,N',N'-Tetramethyl-6-Carboxyrhodamin (TAMRA), VIC, NED oder 5-Carboxy-X-Rhodamin (ROX).

5. Verwendung eines Verfahrens nach einem der Ansprüche 1 bis 4 für die Analyse von C. difficile-Proben, insbesondere von Proben von Patienten mit Diarrhöe, insbesondere von einer Vielzahl solcher Patienten.

6. Kit umfassend eines PCR-Kits zur Analyse von Polymorphismen in der 16s-23s "intergenic spacer"-Region von C. difficile und einer Kapillargelelektrophorese-Vorrichtung, wobei das PCR-Kit einen 16s Primer entsprechend den Basen 1450 bis 1501 des 16s ribosomalen RNA-Gens von C. difficile enthält und einen 23s Primer entsprechend den Basen 1 bis 50 des 23s ribosomalen RNA-Gens von C. difficile, **dadurch gekennzeichnet, dass** der 16s Primer markiert ist und der 23s Primer nicht markiert ist.

## Revendications

1. Procédé d'analyse par réaction en chaîne par polymérase (PCR) de polymorphismes dans la région espaceur intergénique 16s-23s de *Clostridium difficile* ("ribotypage par PCR de *Clostridium difficile*") comprenant l'utilisation d'une amorce 16s correspondant aux bases 1450 à 1501 du gène de l'ARN ribosomique 16s de *C. difficile* et d'une amorce 23s correspondant aux bases 1 à 50 du gène de l'ARN ribosomique 23s de *C. difficile,* **caractérisé en ce que** l'on effectue la PCR avec une amorce 16s marquée et une amorce 23s non marquée et **en ce que** l'on sépare les molécules d'ADN produites par la PCR selon leur taille par électrophorèse capillaire sur gel.

2. Procédé selon la revendication 1, **caractérisé en ce que** l'on effectue la PCR avec les amorces 5'-GTGCGGCTGGATCACCTCCT et 5'-CCCTGCACCCTTAATAACTTGACC.

3. Procédé selon la revendication 1 ou 2, **caractérisé en ce que** l'on effectue la PCR en moins de 30 cycles, de préférence en 18 à 28 cycles, en particulier en 20 à 25 cycles.

4. Procédé selon l'une quelconque des revendications 1 à 3, **caractérisé en ce que** l'amorce 16s est marquée par fluorescence, de préférence avec de la carboxyfluorescéine (FAM), hexachlorofluorescéine (HEX), tétrachlorofluorescéine (TET), N,N,N',N'-tétraméthyl-6-carboxyrhodamine (TAMRA), VIC, NED ou 5-carboxy-X-rhodamine (ROX).

5. Utilisation d'un procédé selon l'une quelconque des revendications 1 à 4 pour l'analyse d'échantillons de *C. difficile,* en particulier d'échantillons de patients souffrant de diarrhée, en particulier d'une pluralité de ces patients.

6. Kit comprenant un kit de PCR pour l'analyse de polymorphismes dans la région espaceur intergénique 16s-23s de *Clostridium difficile* et un appareil d'électrophorèse capillaire sur gel, dans lequel le kit de PCR contient une amorce 16s correspondant aux bases 1450 à 1501 du gène de l'ARN ribosomique 16s de *C. difficile* et une amorce 23s correspondant aux bases 1 à 50 du gène de l'ARN ribosomique 23s de *C. difficile,* **caractérisé en ce que** l'amorce 16s est marquée et l'amorce 23s n'est pas marquée.
